# FASCICULE DE BREVET EUROPEEN

(11) **EP 1 795 173 B1**
(45) Date de publication et mention de la délivrance du brevet: **08.04.2009**
(21) Numéro de dépôt: 06124927.2
(22) Date de dépôt: 28.11.2006
(51) Int. Cl.: A61K 8/02, A61K 8/81, A61K 8/44, A61Q 19/10, A61Q 1/14

(54) **Article cosmétique soluble moussant**
Schaumbildendes, lösliches, kosmetisches Produkt
Soluble foaming cosmetic article

(30) Priorité: 07.12.2005 FR 0553751
(43) Date de publication de la demande: 13.06.2007
(73) Titulaire: L'Oréal, 75008 Paris (FR)
(72) Inventeur: Aubrun-Sonneville, Odile, 92160 Antony (FR); Bordeaux, Dominique, 91450 Soisy sur Seine (FR)
(74) Mandataire: Rasson, Catherine

(56) Documents cités:
- EP-A- 1 502 875
- WO-A-20/05003423
- FR-A- 2 819 416
- US-A- 4 416 791

## Description

La présente invention concerne un article soluble moussant, comprenant un support soluble dans l'eau et au moins un tensioactif à base d'acylaminoacide, et l'utilisation du dit article pour le nettoyage de la peau et/ou des cheveux et/ou le démaquillage de la peau et/ou comme produit de gommage de la peau. L'article est partiellement ou entièrement soluble dans l'eau.

Les produits moussants sont généralement difficiles à protéger sur le plan microbiologique car les tensioactifs ont tendance à inhiber l'activité de certains antimicrobiens. Pour cette raison, il peut être avantageux de disposer de produits moussants anhydres qui ne contiennent pas d'eau ou très peu d'eau, ce qui permet d'éviter les problèmes microbiologiques. Les produits riches en glycols constituent une solution possible pour pallier ce problème. Toutefois, les qualités cosmétiques de ces produits sont souvent médiocres car la présence de glycols donne un effet collant à la texture et inhibe le développement de la mousse.

Une autre solution consiste à utiliser des produits sous forme de poudre. Toutefois, ces produits ne sont pas pratiques à utiliser car il est difficile de doser la quantité à utiliser. Par ailleurs, la volatilité de certaines poudres peut causer des problèmes d'irritations du nez et de la gorge. Enfin, ces produits en poudre, s'ils sont présentés tels quels, renvoient à l'univers de la lessive, ce qui peut créer chez le consommateur de produits cosmétiques un a priori négatif.

Le document WO-A-2005/003423 décrit des articles à base de fibres solubles en PVA qui peuvent être imprégnés avec des tensioactifs anioniques et servent comme der articles de nettoyage ou démaquillantes.

Par ailleurs, le document WO-A-2005/060931 décrit des films solubles contenant des tensioactifs moussants ; ce sont des lingettes obtenues à partir de polymères filmogènes et d'agents nettoyants. Ces lingettes sont obtenues en solubilisant le polymère dans l'eau, puis en appliquant le gel formé en couche mince sur un support pour le faire sécher et former un film fin. L'agent nettoyant est ensuite appliqué sur le film par différentes techniques possibles. La lingette obtenue se solubilise en moins de 2 minutes au contact de l'eau. Toutefois, cette technologie impose une limitation assez importante sur la quantité d'agent nettoyant utilisable (au maximum 30%) comparativement à la quantité de polymère qui est largement majoritaire puisqu'elle représente le plus souvent de 70 à 99% du film fin obtenu, ce qui limite les qualités d'usage du produit, en particulier son pouvoir moussant.

Il subsiste donc le besoin de compositions moussantes n'ayant pas les inconvénients de celles de l'art antérieur, et notamment présentant une bonne conservation microbiologique tout en étant facile et agréable à utiliser, sans avoir d'effet collant, et contenant suffisamment de tensioactif moussant pour avoir un bon effet moussant, tout en présentant un bon développement de la mousse.

La présente demande répond à ce besoin. En effet, la demanderesse a trouvé de manière surprenante qu'il était possible d'utiliser des tensioactifs moussants pulvérulents particuliers dans un support soluble dans l'eau, pour obtenir des produits moussants se présentant généralement sous forme d'unidoses prêtes à l'emploi, ayant de bonnes qualités cosmétiques et de bonnes propriétés moussantes. Ces tensioactifs particuliers qui sont des acylaminoacides ou des dérivés d'acylaminoacides permettent d'obtenir des articles nettoyants qui se solubilisent facilement dans l'eau en donnant un lait agréable à utiliser, alors que d'autres tensioactifs se solubilisent mal ou bien ils se solubilisent bien mais donnent des pâtes gluantes ou agglomérées peu agréables à utiliser. Seuls, les acylaminoacides et leurs dérivés permettent d'atteindre le but de l'invention en permettant d'obtenir un article soluble qui mousse bien et est agréable à utiliser.

Les tensioactifs de type acylaminoacide sont décrits dans FA-A-2 819 416 et EP-A-1 502 875 comme des tensioactifs moussants pour des compositions de nettoyage ou démaquillantes.

Ainsi, l'invention réside, selon l'un de ses aspects, dans un article pour application topique, notamment cosmétique ou dermatologique comportant :
- un support sous forme d'au moins une nappe comprenant des fibres solubles dans l'eau à une température inférieure ou égale à 30°C, et
- une composition portée par le support, comprenant au moins un tensioactif moussant choisi parmi les acylaminoacides et leurs dérivés.

Selon un mode de réalisation particulier, est exclu de l'invention, un article comportant une composition contenant 50 % en poids de Sodium laureth sulfate à 70% dans l'eau (Texapon N702 de chez Cognis), 24,9 % en poids de Disodium cocoamphodiacétate à 39% dans l'eau saumurée (11 % chlorure de sodium) (Miranol C2M de chez Rhodia), 16,9 % en poids de Lauroyl sarcosinate de sodium à 90% dans l'eau (Sarkosyl NL97 de chez Ciba Geigy), 3 % en poids de parfum, 5 % en poids de glycérol, 0,3 % en poids de conservateurs.

On entend par « article cosmétique ou dermatologique», un produit cosmétique ou dermatologique comprenant un support solide comportant une composition portée par ce support.

On entend dans la présente demande par « portée par le support », le fait que la composition peut être soit mise sur le support soit introduite dans la cavité formée par le support quand celui-ci comporte au moins deux nappes formant une cavité.

Par ailleurs, on entend ici par « une composition comprenant au moins un tensioactif moussant choisi parmi les acylaminoacides et leurs dérivés », une composition qui peut être constituée uniquement d'un ou plusieurs tensioactifs moussants choisis parmi les acylaminoacides et leurs dérivés, ou bien une composition contenant au moins un tensioactif moussant choisi parmi les acylaminoacides et leurs dérivés, et un ou plusieurs autres composés. Ainsi, le ou les acylaminoacides ou dérivés d'acylaminoacide peuvent constituer les seuls composés de la composition portée par le support.

En outre, on entend par « température inférieure ou égale à 30°C », une température ne dépassant pas 30°C mais n'étant pas inférieure à 0°C, par exemple allant de plus de 0°C à 30°C, mieux de 5°C à 30°C et encore mieux de 10°C à 30°C.

Cet article se présente de préférence sous forme de doses uniques, ce qui offre les avantages suivants :
- Le produit est pratique à utiliser,
- Le produit peut facilement être emporté sur soi ou en voyage sans crainte de le renverser ou de le voir couler,
- le produit est exempt de conservateur,
- le produit peut être entièrement soluble dans l'eau, ce qui limite la quantité de déchets et est donc bon pour l'environnement.

Les termes «nappe» et «couche» doivent être considérés comme synonymes dans la présente demande. Le support de la présente invention se présente sous forme d'une ou plusieurs nappes de fibres, ce qui est différent des films fins hydrosolubles qui ne sont pas sous forme de nappes de fibres. Par rapport à ces films fins hydrosolubles, les supports à base de nappes de fibres hydrosolubles selon l'invention présentent l'avantage de permettre l'incorporation de constituants incompatibles, d'être plus simples à mettre oeuvre car ils ne nécessitent pas de prémélange ni de mise en solution des composants, ni de chauffage pour évaporer le solvant, le procédé étant également plus rapide et moins coûteux. En outre, les supports selon l'invention ont l'avantage de permettre une plus grande diversité dans le choix de la forme et de l'aspect de l'article car la nappe de fibres peut avoir une épaisseur et une densité variables donnant accès à une grande variété de forme et de taille, alors que le film fin est difficile à sécher si l'épaisseur est trop grande, et il est fragile et difficile à manipuler si la taille est trop grande.

Selon un mode préféré de réalisation de l'invention, l'article se présente sous la forme d'au moins deux nappes définissant entre elles une cavité, l'une au moins des nappes comprenant des fibres solubles dans l'eau à une température inférieure ou égale à 30°C,
- la cavité contenant une composition contenant au moins un tensioactif moussant choisi parmi les acylaminoacides et leurs dérivés.

Les nappes sont assemblées à leur périphérie et forment ainsi une cavité permettant d'introduire la composition contenant le ou les tensioactifs moussants utilisés selon l'invention.

Les nappes peuvent être formées entièrement de fibres solubles dans l'eau ou bien l'une des nappes peut être constituée entièrement de fibres solubles et l'autre nappe peut être constituée de fibres insolubles ou à la fois de fibres solubles et de fibres insolubles dans l'eau, ou bien les deux nappes peuvent être constituées à la fois de fibres solubles et de fibres insolubles,

Selon un mode préféré de réalisation, au moins une des nappes est constituée exclusivement de fibres solubles dans l'eau.

Par humidification ou dissolution de l'article selon l'invention dans l'eau ou dans une composition aqueuse telle qu'une lotion, on obtient une composition moussante pour application topique, notamment cosmétique ou dermatologique, utile pour le nettoyage de la peau ou des cheveux. Si le support ne comporte que des fibres solubles, il va être entièrement dissous dans l'eau, et s'il comporte des fibres solubles et des fibres insolubles, ces dernières vont rester sous forme solide après solubilisation de l'article, et l'article pourra alors par exemple constituer un scrub doux. Quand l'article est dissous dans une lotion, celle-ci peut éventuellement contenir des actifs qui seront donc mélangés aux constituants de l'article au moment de la dissolution de ce dernier.

Ainsi, l'invention a encore pour objet, selon un autre de ses aspects, une composition pour application topique, obtenue par la dissolution dans l'eau, d'un article tel que défini ci-dessus, c'est-à-dire une composition obtenue par dissolution dans l'eau, d'un support sous forme d'au moins une nappe comprenant des fibres solubles dans l'eau à une température inférieure ou égale à 30°C, le dit support portant une composition contenant au moins un tensioactif moussant choisi parmi les acylaminoacides et leurs dérivés.

La température de dissolution de l'article dans l'eau est généralement la température ambiante (20 à 30°C) mais peut être supérieure à la température ambiante si l'on le souhaite selon l'utilisation envisagée.

L'invention a encore pour objet, selon un autre de ses aspects, un procédé cosmétique de nettoyage d'une matière kératinique telle que la peau, les cheveux, les muqueuses, et notamment un procédé cosmétique de nettoyage de la peau, comportant :
- la formation d'une composition cosmétique par dissolution dans l'eau, d'un support comportant au moins une nappe comprenant des fibres solubles dans l'eau à une température inférieure ou égale à 30°C, et portant au moins un tensioactif moussant choisi parmi les acylaminoacides et leurs dérivés,
- l'application de la composition ainsi formée sur la matière kératinique.

Le nettoyage inclut le démaquillage de la peau.

Par «dissolution dans l'eau à une température inférieure ou égale à 30°C », il faut comprendre une solubilisation dans l'eau à une température allant jusqu'à 30°C avec l'aide d'une agitation manuelle et/ou d'une friction du support le cas échéant, dans un laps de temps typiquement inférieur à 5 mn, de préférence inférieur à 1 mn, de préférence inférieur à 30 secondes. L'invention n'exclut pas qu'une eau de température supérieure à 30 °C soit utilisée pour dissoudre le support.

L'article selon l'invention étant destinée à une application topique, il comprend un milieu physiologiquement acceptable. On entend par « milieu physiologiquement acceptable » un milieu compatible avec les matières kératiniques telles que la peau, les lèvres, les ongles, le cuir chevelu et/ou les cheveux. Il en est de même du support, ainsi que de la composition portée par le support.

L'article selon l'invention ne contient pas d'adhésif, mais il peut adhérer à la peau quand il est humidifié.

En outre, cet article est flexible, c'est-à-dire souple. Par « souple », il faut comprendre un article pouvant être comprimé ou pouvant fléchir sans se rompre, capable de s'adapter aux reliefs du corps humain. Un article souple réalisé sous la forme d'une nappe fibreuse peut, dans certains exemples de réalisation, être replié sur lui-même au moins une fois sans se casser en deux morceaux.

Cet article est généralement à usage unique.

Par ailleurs, l'article est généralement sec au toucher avant l'utilisation.

Après sa fabrication, l'article peut être par exemple conditionné en vrac dans une boîte ou dans un emballage individuel. Le cas échéant, les articles sont conditionnés en chapelet. Les articles peuvent encore être repliés sur eux-mêmes et intercalés, de telle sorte que le retrait d'un article amène le suivant dans une configuration facilitant sa préhension.

Ainsi, l'invention a encore pour objet, selon un autre de ses aspects, un ensemble comportant :
- un emballage,
- au moins un article tel que défini plus haut.

L'invention offre ainsi de nouvelles possibilités pour le conditionnement et la formulation des produits cosmétiques moussants.

Dans un exemple de mise en oeuvre de l'invention, on passe rapidement l'article sous l'eau, puis on le place au creux de la main pendant quelques secondes (par exemple 5 secondes) puis on le masse pour développer la mousse en ajoutant si nécessaire de l'eau par petites quantités pour augmenter le volume de mousse. Ensuite, on applique la mousse sur la peau, par exemple la peau du visage préalablement humidifiée ou non, et on procède au nettoyage de la peau par massage et on la rince à l'eau claire puis on la sèche.

Au lieu d'utiliser de l'eau, on peut utiliser une composition cosmétique aqueuse pour hydrater l'article peut également être réhydrater avec une composition aqueuse, c'est-à-dire contenant au moins 50 % en poids d'eau par rapport au poids total de la composition, cette composition pouvant se présenter sous forme de lotion, de lait, de crème ou d'un gel.

### Support

Le support se présente sous forme d'une nappe comprenant des fibres hydrosolubles, c'est-à-dire des fibres solubles dans l'eau à une température inférieure ou égale à 30°C, de préférence solubles dans l'eau à une température inférieure ou égale à 20°C, c'est-à-dire ayant une température de dissolution dans l'eau allant de plus de 0°C à 30°C, de préférence de plus de 0°C à 20°C, et par exemple de 5°C à 30°C, et mieux de 5 à 20°C.

Le support peut être sensiblement non rétractable une fois mouillé.

Le support peut avoir toute forme appropriée pour l'utilisation visée, par exemple une forme rectangulaire, ronde ou ovale, et il a de préférence des dimensions permettant sa préhension entre deux doigts au moins. Ainsi, le support peut avoir par exemple une forme ovoïde d'environ 2 à 10 cm de long et d'environ 0,5 à 4 cm de large, ou une forme de disque d'environ 2 à 10 cm de diamètre, ou une forme de carré d'environ 5 à 15 cm de côté, ou une forme de rectangle d'une longueur d'environ 5 à 15 cm, étant entendu qu'il peut avoir toute autre forme et dimension appropriées pour l'utilisation recherchée.

Le support peut former par exemple un coussinet, un masque, un patch, une charlotte, un doigt de gant ou un gant, une nappe à découper, une lingette, un disque, un oval ou un rectangle. En outre, le support peut présenter une forme qui dépend de la région du corps à nettoyer.

Le support peut présenter une forme aplatie ou une forme non aplatie, présentant par exemple l'aspect d'un bloc formé d'un amas globulaire de fibres hydrosolubles compactées, incorporant une composition contenant les tensioactifs utilisés selon l'invention.

Les fibres du support sont généralement enchevêtrées pour former la nappe de fibres. Comme indiqué plus haut, on entend par « nappe comprenant des fibres solubles dans l'eau », une nappe pouvant être entièrement constituée de fibres solubles dans l'eau ou une nappe pouvant comporter à la fois des fibres solubles dans l'eau et des fibres insolubles dans l'eau, les fibres solubles devant être en plus grande quantité que les fibres insolubles. La nappe de fibres doit comporter au moins 60 % en poids de fibres solubles, de préférence au moins 70 % et mieux au moins 80 % en poids par rapport au poids total des fibres du support. Elle peut ainsi comporter, par exemple, plus de 95 % en poids, voire plus de 99 % en poids et même 100 % en poids de fibres hydrosolubles par rapport au poids total des fibres du support. Ainsi, le support peut être constitué entièrement de nappes de fibres solubles ou il peut être constituée de nappes comportant un mélange de fibres solubles et de fibres insolubles, les fibres insolubles étant selon la définition de la présente invention, des fibres qui ne sont pas solubles dans l'eau à une température inférieure ou égale à 30°C. Le fait d'avoir des fibres insolubles peut permettre d'avoir un produit moussant qui soit en même temps un produit de gommage doux (scrub), les fibres insolubles constituant le composé exfoliant.

Ainsi, le support peut être formé de deux nappes constituées de fibres solubles dans l'eau, ou encore d'une nappe constituée de fibres solubles dans l'eau et d'une nappe comprenant à la fois de fibres solubles et de fibres insolubles, ou bien aussi d'une nappe constituée de fibres solubles dans l'eau et d'une nappe constituée de fibres insolubles dans l'eau, ou bien de deux nappes comprenant à la fois de fibres solubles et de fibres insolubles. Il peut y avoir aussi plus de deux nappes.

Selon un mode préféré de réalisation de l'invention, le support est dépourvu de fibres insolubles dans l'eau et il est composé uniquement de fibres solubles de l'eau, de sorte qu'il soit entièrement soluble dans l'eau.

Les fibres solubles peuvent être en tout matériau soluble susceptible d'étre filé en fibres. De préférence, les fibres solubles dans l'eau sont réalisées avec de l'alcool polyvinylique (PVA) selon un procédé qui leur confère la solubilité recherchée, le PVA pouvant avoir plusieurs grades de polymérisation.

Des fibres de PVA solubles dans l'eau à une température inférieure ou égale à 30°C sont commercialisées par la société japonaise KURARAY sous la dénomination commerciale KURALON K-II WN2. Le procédé de fabrication de ces fibres comporte la préparation d'une solution à filer par dissolution d'un polymère à base de PVA soluble dans l'eau, dans un premier solvant organique, le filage de la solution dans un second solvant organique pour obtenir des filaments solidifiés et l'étirage humide des filaments dont on enlève le premier solvant puis que l'on sèche et que l'on soumet à un traitement thermique. La section de ces fibres peut être sensiblement circulaire. Ces fibres ont une résistance à la traction d'au moins 2,7 g/dtex (3 g/d). La demande EP-A-0 636 716 décrit de telles fibres hydrosolubles à base de PVA et leur procédé de fabrication.

L'invention n'est pas limitée à l'emploi de PVA, et on peut utiliser aussi des fibres réalisées dans d'autres matériaux hydrosolubles sous réserve que ces matériaux se dissolvent dans de l'eau ayant la température recherchée, par exemple des fibres de polysaccharides commercialisées sous la dénomination LYSORB par la société LYSAC TECHNOLOGIES, INC ou des fibres à base de polymères polyholosides comme le glucomannane ou l'amidon.

La nappe de fibres peut comporter, le cas échéant, un mélange de différentes fibres solubles dans l'eau à des températures différentes (jusqu'à 30°C).

Les fibres peuvent être composites, et elles peuvent comporter par exemple un coeur et une gaine n'ayant pas la même nature, par exemple formés de différents grades de PVA.

Quand la nappe de fibres contient des fibres insolubles, celles-ci peuvent être en toute matière habituellement utilisée comme fibres insolubles ; ce peut être par exemple des fibres de soie, de coton, de laine, de lin, de cellulose extraites notamment du bois, des légumes ou des algues, de polyamide (Nylon^{®}), d'acide polylactique, de cellulose modifiée (rayonne, viscose, acétate notamment d'acétate de rayonne), de poly-p-phénylène téréphtalamide notamment de Kevlar^{®}, en acrylique notamment de polyméthacrylate de méthyle ou de poly 2-hydroxyéthyl méthacrylate, de polyoléfine et notamment de polyéthylène ou de polypropylène, de verre, de silice, d'aramide, de carbone notamment sous forme graphite, de Téflon^{®}, de collagène insoluble, de polyesters, de polychlorure de vinyle ou de vinylidène, d'alcool polyvinylique, de polyacrylonitrile, de chitosane, de polyuréthane, de polyéthylène téréphtalate, de fibres formées d'un mélange des composés mentionnés ci-dessus, comme des fibres de polyamide/polyester ou de viscose/polester. Les non tissés sont décrits de façon générale dans RIEDEL «Nonwoven Bonding Methods & Materials», Nonwoven World (1987), incorporé ici par référence.

Dans un exemple particulier de réalisation de l'invention, la nappe du support est un non-tissé, comportant des fibres hydrosolubles, seules ou en mélange avec des fibres insolubles comme indiqué plus haut, avec au plus de 40% en poids de fibres insolubles par rapport au poids total des fibres du support. De préférence, le non-tissé est constitué de fibres hydrosolubles, c'est-à-dire qu'il ne contient pas de fibres insolubles.

Par ailleurs, le support peut comporter en outre au moins une couche d'un substrat insoluble dans l'eau, c'est-à-dire ne comportant que des fibres insolubles, et dans cet exemple de réalisation,le support comprend une nappe d'un non-tissé constitué de fibres solubles dans l'eau à une température inférieure ou égale à 30°C, et une nappe d'un non-tissé constitué de fibres insolubles dans l'eau.

Quand le support ne comporte qu'une seule nappe de fibres, la composition contenant le ou les tensioactifs à base d'acylaminoacides peut être déposée sur les deux faces du support ou sur une seule face, l'autre face du support pouvant alors être utilisée par exemple pour la préhension de l'article.

Quand le support selon la présente invention comporte deux nappes, il peut s'agir notamment de deux nappes de non tissé, tous les modes de réalisation décrits ci-dessous pouvant être utilisés, les nappes pouvant contenir ou non des fibres insolubles, et même une des nappes pouvant être constituée uniquement de fibres insolubles, du moment que l'autre nappe contient des fibres solubles.

Selon un mode particulier de réalisation de l'invention, chacune des nappes est un non-tissé constitué de fibres solubles à une température inférieure ou égale à 30°C, c'est-à-dire que les nappes ne comportent que des fibres hydrosolubles.

Selon un autre mode de réalisation, une des nappes est entièrement soluble dans l'eau et est un non-tissé constitué de fibres solubles à une température inférieure ou égale à 30°C, et l'autre nappe est insoluble et est est un non-tissé constitué de fibres insolubles.

Selon encore un autre mode de réalisation, le support comporte deux nappes contenant des fibres solubles ou partiellement solubles avec au plus 40 % de fibres insolubles, et en outre une nappe constituée de fibres insolubles, constituant un substrat insoluble. Ainsi, le support peut comporter au moins une couche d'un substrat insoluble dans l'eau, c'est-à-dire ne comportant que des fibres insolubles. Dans un exemple particulier de ce mode de réalisation, le support comprend une nappe soluble d'un non-tissé constitué de fibres solubles dans l'eau à une température inférieure ou égale à 30°C, et une nappe insoluble d'un non-tissé constitué de fibres insolubles dans l'eau.

Une structure multicouche avec au moins une couche formée d'un substrat insoluble dans l'eau peut par exemple être utile pour réaliser un article comportant un support en forme de doigt de gant. La couche formée de fibres hydrosolubles est située à l'extérieur de l'article, étant destinée à se solubiliser lors de l'utilisation, après avoir été mouillée ou en venant au contact d'une région mouillée du corps.

Pour fabriquer les nappes en non-tissé, quelles soient solubles ou insolubles, toutes les techniques appropriées de constitution d'un non-tissé à partir de fibres peuvent être utilisées. Par exemple, les fibres peuvent être formées par extrusion et déposées sur un convoyeur pour former une nappe de fibres qui est ensuite consolidée par une technique classique de liage de fibres, telle que par exemple l'aiguilletage, le liage à chaud, le calandrage ou le liage par jets d'air chaud (en anglais *air through bonding*)*,* technique dans laquelle la nappe passe dans un tunnel où est insufflé de l'air chaud. Cette dernière technique est avantageusement utilisée lorsque la nappe est constituée de fibres bicomposant, par exemple des fibres comprenant au moins deux grades d'alcool polyvinylique (PVA), dont les points de fusion ou de ramollissement sont différents, ces fibres étant par exemple co-extrudées de manière à ce que la fibre soit constituée d'au moins un premier grade localisé au coeur de la fibre et d'au moins un deuxième grade localisé en périphérie de la fibre, sous la forme d'une gaine. Le liage des fibres peut être plus facile lorsque la gaine présente un point de fusion plus faible que le coeur.

La nappe de fibres peut encore être formée par cardage de fibres découpées à une longueur de 10 à 50 mm, puis dépôt des fibres sur un convoyeur où la nappe peut ensuite être consolidée par une technique de liage telle que décrite ci-dessus.

Lorsque le support comporte plusieurs couches, que celles-ci soient toutes réalisées avec des fibres hydrosolubles ou non, les différentes couches peuvent être assemblées de multiples manières, par exemple par soudage, collage ou couture, et ces couches peuvent constituer le cas échéant une ou plusieurs cavités contenant une ou plusieurs compositions cosmétiques ou dermatologiques ou plusieurs composants d'une même composition cosmétique à mélanger extemporanément. Lors d'un assemblage par couture, un fil lui-même hydrosoluble peut être utilisé, le cas échéant.

Quand le support comporte plusieurs nappes de non tissé, celles-ci peuvent être assemblées notamment par thermosoudage à leur périphérie de manière à constituer un coussinet capable de retenir dans une cavité intérieure, une composition contenant au moins un tensioactif moussant dérivé d'acylaminoacide.

Selon un autre aspect de l'invention, le support est dépourvu d'adhésif, notamment d'adhésif sensible à la pression.

La densité du support pourra dépendre des applications. Le support peut présenter, par exemple, une densité inférieure ou égale à 0,1 g/cm³ ou bien supérieure à 0,1g/ cm³. Selon un mode préféré de réalisation de l'invention, le support a une densité inférieure ou égale à 0,1 g/cm³, mieux allant de 0,01 g/cm³ à 0,1 g/cm³, ce qui permet d'avoir un support très aéré, qui, de ce fait, se dissout dans l'eau plus facilement.

La composition portée par le support représente entre 10 et 1000 % en poids et mieux entre 10 et 500 % en poids par rapport au poids du support, en entendant ici par «poids du support», le poids du support seul, sans le poids de la composition. Si la composition ne contient que les acylaminoacides et leurs dérivés, ce sont ces composés qui peuvent représenter entre 10 et 1000 % en poids par rapport au poids du support, et de préférence entre 10 et 500 % en poids par rapport au poids du support.

### Tensioactifs acylaminoacides

La composition contenant les tensioactifs moussants peut ne comporter que le ou les tensioactifs moussants à base d'acylaminoacides ou leurs dérivés, ou bien elle peut comporter ces tensioactifs en mélange avec d'autres composés, par exemple d'autres tensioactifs moussants et des additifs.

L'article selon l'invention contient un ou plusieurs tensioactifs moussants choisis parmi les acylaminoacides et leurs dérivés. Ces tensioactifs sont notamment choisis parmi les sels alcalins de N-acylaminoacides. Comme sels alcalins d'acylaminoacides, on peut citer notamment les sels d'acylalaninates, les sels d'acylglutamates, les sels d'acylaspartates, les sels d'acylglycinates, les sels d'acylsarcosinates, et leurs mélanges.

Les tensioactifs préférés sont ceux en poudre, tels que par exemple le sodium lauroyl glutamate commercialisé sous la dénomination Amisoft LS 11 par la société Ajinomoto ; le myristoyl glutamate monosodique commercialisé sous la dénomination Acylglutamate MS 11 par la société Ajinomoto ; le lauroyl methyl beta-alanine (forme acide) commercialisé sous la dénomination LMA-H par la société Mitsui Toatsu ; le N-lauroyl-N-hydroxyethyl-beta-alanine commercialisée sous la dénomination LHEA par la société Mitsui Toatsu ; le sodium cocoyl glycinate commercialisé sous la dénomination Amilite GCS-11(F) par la société Ajinomoto.

De manière particulièrement préférée, les acylaminoacides et leurs dérivés sont choisis parmi les acylglutamates tels que le lauroylglutamate de sodium, et le myristoylglutamate de sodium, les acylglycinates tels que le cocoylglycinate de sodium, et leurs mélanges.

Selon un mode de réalisation particulier, le tensioactif acylaminoacide n'est pas le lauroyl sarcosinate dans une composition contenant 50 % en poids de Sodium laureth sulfate à 70% dans l'eau (Texapon N702 de chez Cognis), 24,9 % en poids de Disodium cocoamphodiacétate à 39% dans l'eau saumurée (11 % chlorure de sodium) (Miranol C2M de chez Rhodia), 16,9 % en poids de Lauroyl sarcosinate de sodium à 90% dans l'eau (Sarkosyl NL97 de chez Ciba Geigy), 3 % en poids de parfum, 5 % en poids de glycérol, 0,3 % en poids de conservateurs.

Les tensioactifs moussants utilisés selon l'invention peuvent être mis tels quels sur le support ou être mélangés à d'autres composés.

Le ou les acylaminoacides et leurs dérivés peuvent représenter 100 % de la composition portée par le support. Ils peuvent être présents en une quantité allant par exemple de 2 à 100 % et mieux de 10 à 100 % du poids total de la composition portée par le support, et de préférence de 20 à 100 % en poids par rapport au poids total de la composition portée par le support. On entend dans la demande par « % en poids par rapport au poids total de la composition » le pourcentage en poids par rapport au poids total de la composition portée par le support (et non par rapport au poids de l'article comprenant support et composition).

### Compositions

Les compositions contenant le ou les tensioactifs moussants à base d'acylaminoacides sont des compositions anhydres. Elles sont de préférence sous forme pulvérulente ou pâteuse, et plus préférentiellement sous forme pulvérulente. Ce sont des compositions adaptées à une application topique, notamment des compositions cosmétiques ou dermatologiques.

Ainsi, les compositions utilisables dans l'invention peuvent être par exemple des émulsions lyophilisées ou atomisées, telles que celles décrites dans le document FR-A-2,727,312 ou celles à base d'amidon modifié, décrites dans les documents EP-A-0 938 892 et EP-A-0 925 777. Ces émulsions sont obtenues par lyophilisation ou atomisation d'une émulsion H/E contenant une phase pulvérulente, conduisant à des laits ou des crèmes par mélange avec l'eau lors de leur utilisation.

Elles peuvent aussi être obtenues par simple mélange des constituants, ceux-ci étant de préférence en poudre.

La composition peut ne contenir que les tensioactifs moussants à base d'acylaminoacides qui représentent alors 100 % du poids de la composition.

Selon les constituants des compositions utilisées, l'article se transforme en mousse, ou en lait moussant ou en crème moussante.

La composition peut éventuellement contenir une certaine quantité d'eau au moment de son imprégnation sur le support. Toutefois, de manière à éviter sa solubilisation prématurée, l'eau introduite sur le support lors de son imprégnation doit être éliminée par les moyens classiquement utilisés pour la déshydratation des compositions contenant de l'eau, comme par exemple le chauffage. Cependant, la composition peut contenir une certaine quantité d'eau qui est généralement de l'eau liée et qui peut provenir notamment des matières premières hygroscopiques qui contiennent de l'eau, telles que les amidons La quantité d'eau finale dans la composition présente sur l'article est d'au maximum 20 % en poids et de préférence au maximum 10 % en poids par rapport au poids total de la composition.

Lorsque la composition doit être déposée sur le support par l'utilisateur lui-même, la composition et le support peuvent être proposés ensemble, sous la forme d'un kit, par exemple. La composition est par exemple livrée en une quantité suffisante pour permettre d'en distribuer une pluralité de doses sur un ensemble de supports destinés à être utilisés successivement.

### Autres ingrédients

La composition contenue dans le support peut contenir, outre les tensioactifs moussants à base d'acylaminoacides, d'autres ingrédients, notamment d'autres tensioactifs moussants et des additifs usuels dans le domaine cosmétique, tels que des exfoliants, des polymères, des huiles, des particules comme le kaolin. Ces autres ingrédients sont de préférence sous forme pulvérulente ou pâteuse, et il s'agit notamment de tensioactifs se présentant sous forme pulvérulente ou pâteuse, mais si nécessaire, les additifs peuvent être encapsulés ou adsorbés sur des poudres.

### Autres tensioactifs moussants

Comme tensioactifs moussants autres que les acylaminoacides et leurs dérivés, on peut utiliser tous ceux habituellement utilisés dans le domaine cosmétique, ces tensioactifs pouvant être anioniques, non ioniques, cationiques, amphotères ou zwitterioniques.

La quantité totale de tensioactif(s) moussant(s) y compris les acylaminoacides et leurs dérivés peut aller par exemple de 2 à 100 % en poids, de préférence de 10 à 100 % en poids par rapport au poids total de la composition portée par le support.

Comme tensioactif anionique moussant, on peut citer les sels d'acide gras qui constituent les savons et qui sont dérivés d'un acide gras ayant une chaîne alkyle comportant de 6 à 22 atomes de carbone, de préférence de 8 à 18 atomes de carbone, l'agent neutralisant pouvant être une base organique ou minérale telle que la potasse, la soude, la triéthanolamine, la N-methylglucamine, la lysine et l'arginine ; les alkylsulfates et alkylethersulfates ; les sulfonates, et leurs mélanges.

Comme tensioactifs non ioniques, on peut citer par exemple les esters de sucre, les éthers de sucre comme les alkyl polyglucosides (APG), les condensats d'oxydes d'alkylène et d'alkyl phénols, les éthers d'alcool gras et de polyols, et leurs mélanges.

Comme tensioactifs amphotères ou zwitterioniques, on peut citer les bétaïnes et leurs dérivés, les sultaïnes et leurs dérivés, les dérivés imidazolinium, et leurs mélanges.

Les tensioactifs préférés sont ceux en poudre, tels que par exemple le lauryl sulfate de sodium comme le produit commercialisé sous la dénomination Empicol LZ D par la société Allbright & Wilson ou sous la dénomination Tensopol USP97 par la société Tensachem; la cocamidopropylbetaine comme le produit commercialisé sous la dénomination Tegobetain CK D par la société Degussa ; le mélange de laureth sulfate de sodium et de silice, commercialisé sous la dénomination Texapon KE 2713 par la société Cognis ; le disodium cocamido MEA-sulfosuccinate comme le produit commercialisé sous la dénomination Mackanate CM 100 par la société Mac Intyre ; le methyl cocoyl taurate de sodium, comme le produit commercialisé sous la dénomination Tauranol WSP par la société Finetex ; le decyl d-galactoside uronate de sodium comme le produit commercialisé sous la dénomination Decyl d-galactoside uronate de sodium par la société Ard-Soliance ; le sodium cocoyl isethionate comme le produit commercialisé sous la dénomination Jordapon CI P par la société BASF ; le lauryl sulfoacetate de sodium, comme le produit commercialisé sous la dénomination Lathanol LAL poudre par la société Stepan ; le myristate de potassium comme le produit commercialisé sous la dénomination Myristate de potassium (DUB MK) par la société Stearinerie Dubois ; le laurate de potassium comme le produit commercialisé sous la dénomination Laurate de potassium (DUB LK) par la société Stearinerie Dubois, et le laurate de sucrose comme le produit commercialisé sous la dénomination Grilloten LSE 87 par la société Degussa.

### Polymères

La composition portée par le support peut contenir aussi un ou plusieurs polymères, notamment des polymères hydrosolubles. A titre d'exemple de polymères hydrosolubles utilisables dans l'invention, on peut citer les gommes telles que la gomme de guar, la gomme de xanthane, la gomme de carraghénane, la gomme de sclerotium, la gomme de Konjac, la gomme d'adragante, et les dérivés de ces gommes ; les dérivés de cellulose tels que les hydroxyalkylcelluloses, la carboxyméthylcellulose ; les dérivés d'amidon tels que l'hydroxypropyl phosphate d'amidon commercialisé sous la dénomination Structure XL par la société National Starch (nom INCI : Hydroxypropyl Starch Phosphate) ; les pectines ; les polyacrylamides et les copolymères d'acrylamides et notamment les homopolymères et copolymères d'acide 2-acrylamido 2-méthylpropane sulfonique tels que ceux commercialisés sous les dénominations Hostacerin AMPS et Aristoflex par la société Clariant, la gélatine, l'agar-agar, les polymères carboxyvinyliques tels que les produits commercialisés sous les dénominations Carbopol par la société Noveon (nom CTFA : carbomer), les polymères carboxyvinyliques modifiés et notamment les copolymères acrylate/C₁₀-C₃₀-alkylacrylate tels que les produits commercialisés sous les dénominations Pemulen TR1 ou TR2 ou CARBOPOL 1382 par la société Noveon (nom CTFA : Acrylates/C10-30 Alkyl acrylate Crosspolymer), la montmorillonite et le silicate de magnésium et aluminium.

Lorsqu'ils sont présents, la quantité de polymère(s) dans la composition de l'invention peut aller par exemple de 0,1 à 30 % et de préférence de 0,5 à 20 % du poids total de la composition portée par le support

### Exfoliants

La composition peut contenir aussi des exfoliants, notamment pour constituer une composition de gommage ou un scrub pour le visage ou le corps. Comme exfoliants, on peut citer par exemple des particules exfoliantes ou gommantes d'origines minérale, végétale ou organique. Ainsi, on peut utiliser par exemple des billes ou de la poudre de polyéthylène, de la poudre de nylon, de la poudre de polychlorure de vinyle, de la pierre ponce, des broyats de noyaux d'abricots ou de coques de noix, de la sciure de bois, des billes de verre, l'alumine, et leurs mélanges. Par ailleurs, comme indiqué plus haut, l'exfoliant peut être constitué de fibres insolubles incluses dans la nappe de fibres du support.

Les particules exfoliantes peuvent être présentes en une quantité allant par exemple de 0,5 à 40 % en poids, de préférence de 1 à 20 % en poids et mieux de 1 à 10 % en poids par rapport au poids total de la composition. Quand la composition contient des particules exfoliantes, l'article obtenu peut être utilisé notamment pour le gommage de la peau du visage ou du corps.

### Additifs

La composition de l'invention peut contenir un ou plusieurs additifs, notamment ceux qui sont anhydres ou sous forme solide (poudre), choisis parmi ceux généralement utilisés dans les domaines cosmétique et dermatologique, tels que, par exemple, les séquestrants, les parfums, les antioxydants, les actifs, les conservateurs, les matières colorantes (comme les pigments et les colorants hydrophiles) et les charges minérales et/ou les charges organiques telles que l'amidon modifié comme celui commercialisé sous la dénomination Dry Flo par la société National Starch.

Les actifs peuvent être choisis notamment parmi les agents kératolytiques, les hydratants, les antimicrobiens, les vitamines, les agents antipelliculaires ou antiséborrhéiques, les agents pour la pousse des cheveux. Selon un mode particulier de réalisation de l'invention, les actifs peuvent être encapsulés ou adsorbés sur des poudres.

Comme hydratants, on peut citer les polyols tels que la glycérine ; les composés agissant sur la fonction barrière, en vue de maintenir l'hydratation du stratum corneum, ou les composés occlusifs, en particulier les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline ; les composés augmentant directement la teneur en eau du stratum corneum, tel que le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, et la N-α-benzoyl-L-arginine ; et leurs mélanges.

Comme agents kératolytiques, on peut citer les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique, et leurs mélanges.

Comme antimicrobiens, on peut citer par exemple le 2,4,4'-trichloro-2'-hydroxy diphényl éther (ou triclosan), le 3,4,4'-trichlorocarbanilide (ou triclocarban) le phénoxyéthanol, le phénoxypropanol, le phénoxyisopropanol, l'hexamidine iséthionate, le métronidazole et ses sels, le miconazole et ses sels, l'itraconazole, le terconazole, l'éconazole, le ketoconazole, le saperconazole, le fluconazole, le clotrimazole, le butoconazole, l'oxiconazole, le sulfaconazole, le sulconazole, le terbinafine, le ciclopiroxe, le ciclopiroxolamine, l'acide undécylenique et ses sels, le peroxyde de benzoyle, l'acide 3-hydroxy benzoïque, l'acide 4-hydroxy benzoïque, l'acide phytique, l'acide N-acétyl-L-cystéine, l'acide lipoïque, l'acide azélaïque et ses sels, l'acide arachidonique, le résorcinol, l'octopirox, l'octoxyglycérine, l'octanoylglycine, le caprylyl glycol, l'acide 10-hydroxy-2-décanoïque, le dichlorophenyl imidazol dioxolan et ses dérivés décrits dans le brevet WO-A-93/18743, le farnesol, les phytosphingosines et leurs mélanges.

Comme agents antipelliculaires, on peut citer par exemple les sels de pyridinethione, notamment les sels de calcium, de magnésium, de barium, de strontium, de zinc, de cadmium, d'étain et de zirconium. ; les dérivés de 1-hydroxy-2-pyrrolidone; le 2,2'-dithio-bis-(pyridine-N-oxyde); les trihalogéno-carbamides ; le triclosan; les composés azotés tels que le climbazole, le kétoconazole, le clotrinazole, l'éconazole, l'isoconazole et le miconazole. ; les polymères antifongiques tels que l'amphotéricine B ou la nystatine ; le sulfure de sélénium ; le soufre sous ses différentes formes ; le sulfure de cadmium ; l'allantoïne ; les goudrons de houille ou de bois et leurs dérivés en particulier l'huile de cade ; l'acide salicylique ; l'acide undécylénique ; l'acide fumarique ; les allylamines telle que la terbinafine.

Comme agents pour la pousse des cheveux, on peut citer notamment le 2,4-diamino 6-pipéridinopyrimidine 3-oxyde ou "minoxidil" décrit dans les brevets US-A-4,139,619 et US-A-4,596,812 ou encore ses nombreux dérivés comme ceux décrits par exemple dans les demandes de brevet EP-A-0353123, EPA-0356271, EP-A-0408442, EP-A-0522964, EP-A-0420707, EP-A-0459890, EP-A-0519819.

Comme vitamines, on peut utiliser les vitamines ou provitamines hydrosolubles ou liposolubles, comme par exemple les vitamines A (rétinol), C (acide ascorbique), B3 ou PP (niacinamide), B5 (panthénol), B6 ou pyridoxine, E (tocophérol), K1, le bêta-carotène, et les dérivés de ces vitamines et notamment leurs esters, et leurs mélanges.

La composition peut contenir aussi des composés lipophiles, par exemple des huiles comme l'huile de vaseline, et des composés siliconés, dans la mesure où ils ne perturbent pas la formation de mousse. Les composés lipophiles sont généralement en une quantité inférieure à 10 % en poids par rapport au poids total de la composition.

Bien entendu, l'homme de l'art veillera à choisir ce ou ces éventuels additifs et/ou leurs quantités de manière telle que les propriétés avantageuses attachées intrinsèquement à la composition conforme à l'invention ne soient pas, ou substantiellement pas, altérées par la ou les adjonctions envisagées.

L'article selon l'invention peut constituer notamment un produit de nettoyage de la peau (du visage et/ou du corps) ou des cheveux (shampooing), et un produit exfoliant pour la peau (du visage et/ou du corps).

Les exemples qui suivent servent à illustrer l'invention sans toutefois présenter un caractère limitatif. Les quantités indiquées sont en % en poids sauf mention contraire, et elles correspondent sauf mention contraire à la quantité de matière première et non à la quantité de matière active. Les noms des composés utilisés sont indiqués en nom CTFA, en nom chimique ou en nom commercial.

### EXEMPLES

L'article utilisé dans les exemples a été réalisé avec un support en fibres Kuralon K-II WN2 à base de PVA, fibres qui sont solubles à une température inférieure ou égale à 20°C. Il a été obtenu en thermosoudant à leur périphérie deux couches de grammage 80 g/m². L'article se présentait sous la forme d'un disque de 3 cm de diamètre, comportant une cavité dans laquelle a été introduite la composition contenant le tensioactif en une quantité de 0,3 g.

### Exemple 1 selon l'invention et exemples comparatifs 1 à 3

| Composition portée par le support | Exemple 1 selon l'invention | Exemple comparatif 1 | Exemple comparatif 2 | Exemple comparatif 3 |
|---|---|---|---|---|
| Sodium lauroyl glutamate (1) | 100 | - | - | - |
| Sodium Cocoyl Isethionate (2) | - | 50 | - | - |
| Potassium myristate | - | 50 | - | - |
| Lauryl sulfate (3) | - | - | 100 | - |
| Cocamido propyl bétaine (4) | - | - | - | 100 |

| Evaluation du produit | | | | |
|---|---|---|---|---|
| Mélange à l'eau | Solubilisation rapide de l'article, donnant un lait blanc | Solubilisation assez rapide de l'article ; mais produit obtenu très épais et très collant | Mauvaise solubilisation et obtention d'une pâte gluante très glaireuse | Agglomération de la poudre, formant des grains se solubilisant lentement dans l'eau |

| Qualité de mousse | | | | |
|---|---|---|---|---|
| Volume | 6,8 | 5,3 | 8,5 | 7 |
| Taille des bulles | 3 | 0,8 | 7,3 | 5 |
| Densité | 7,5 | 7 | 6,8 | 6,5 |

| | | | | |
|---|---|---|---|---|
| (1) ) Amisoft LS 11 (Ajinomoto) (2) Jordapon CIP (BASF) (3) Tensopol USP97 (Tensachem) (4) Tego Bétain CKD (Degussa) | | | | |

Lors de l'utilisation de l'article, on le passe rapidement sous l'eau, puis on le place au creux de la main pendant quelques secondes et on le masse pour développer la mousse en ajoutant si nécessaire de l'eau par petites quantités pour augmenter le volume de mousse. Ensuite, on applique la mousse obtenue sur le visage préalablement humidifié ou non, et on procède au nettoyage de la peau par massage, puis on la rince à l'eau claire et on la sèche.

Les propriétés moussantes de l'article de l'exemple selon l'invention ont été évaluées par un test d'évaluation.

Test d'évaluation : il a été réalisé selon le protocole suivant :
Les mains sont lavées 3 fois au savon de Marseille et soigneusement rincées.
L'eau en excès est éliminée en secouant 3 fois les mains.

L'article est posé dans le creux d'une des mains.

La mousse est formée en ajoutant 2 ml d'eau dans le creux de la main et en cisaillant le produit pendant 10 secondes en faisant glisser une main sur l'autre (environ 20-30 aller et retour), puis en ajoutant encore 2 ml d'eau et en cisaillant de nouveau le produit pendant 10 secondes.

A l'issue de ce protocole, la mousse a été évaluée selon 3 critères :
- Le volume de mousse (note élevée = volume important)
- la taille des bulles (note élevée = mousse grossière, plus la note est faible, plus la taille des bulles est petite)
- la densité de la mousse (note élevée = mousse plus élastique, non coulante)

Les résultats indiqués dans le tableau ci-dessus montrent que l'exemple selon l'invention donne un produit offrant un bon mélange à l'eau et une très bonne qualité de mousse, tandis que les exemples comparatifs contenant des tensioactifs autres que ceux revendiqués conduisent à des produits se mélangeant moins bien à l'eau et/ou offrant des mousses de moins bonne qualité.

### Exemples 2 à 6 selon l'invention :

| Composition portée par le support | Exemple 2 selon l'invention | Exemple 3 selon l'invention | Exemple 4 selon l'invention | Exemple 5 selon l'invention | Exemple 6 selon l'invention |
|---|---|---|---|---|---|
| Sodium lauroyl glutamate (1) | 50 | - | - | 75 | - |
| MyristoyL glutamate monosodique (2) | - | 50 | 65 | | - |
| Sodium Cocoyl Glycinate (3) | - | 50 | - | | 86 |
| Disodium cocamido mea-sulfosuccinate (4) | - | - | 30 | | - |
| Cocamidopropyl bétaine (5) | 50 | - | - | 20 | - |
| Hydroxypropyl Starch Phosphate (6) | | | 5 | | - |
| Poudre de polyethylene (7) | | | | 5 | |
| Amidon modifié (8) | - | | | | 3.5 |
| Huile de vaseline | - | | | | 10.5 |

| | | | | | |
|---|---|---|---|---|---|
| (1) ) Amisoft LS 11 (Ajinomoto) (2) Acylglutamate MS 11 (Ajinomoto) (3) Amilite GCS-11 (F) (Ajinomoto) (4) Mackanate CM 100 (Mac Intyre) (5) Tego Bétain CKD (Degussa) (6) Structure XL (National Starch) (7) Microthene MN 727 (Equistar) (8) C* Flo 06205 (Cerestar) | | | | | |

Il a été trouvé que les exemples 2 à 6 ci-dessus se mélangeaient bien à l'eau et avaient de bonnes propriétés moussantes

## Revendications

1. Article pour application topique comportant :
- un support sous forme d'au moins une nappe comprenant des fibres solubles dans l'eau à une température inférieure ou égale à 30°C, et
- une composition portée par le support, comprenant au moins un tensioactif moussant choisi parmi les acylaminoacides et leurs dérivés.

2. Article selon la revendication 1, **caractérisé en ce que** les fibres solubles dans l'eau à une température inférieure ou égale à 30°C sont réalisées avec de l'alcool polyvinylique.

3. Article selon la revendication 1 ou 2, **caractérisé en ce que** la nappe de fibres est un non-tissé.

4. Article selon l'une quelconque des revendications précédentes, **caractérisé en ce que** la nappe de fibres comprend des fibres insolubles dans l'eau.

5. Article selon la revendication précédente, **caractérisé en ce que** la quantité de fibres insolubles dans l'eau est d'au plus 40 % en poids par rapport au poids total des fibres.

6. Article selon l'une quelconque des revendications précédentes, **caractérisé en ce que** le support comporte au moins deux nappes dont au moins une contient des fibres solubles dans l'eau à une température inférieure ou égale à 30°C.

7. Article selon l'une quelconque des revendications précédentes, **caractérisé en ce qu'**il comporte :
- au moins deux nappes définissant entre elles une cavité, l'une au moins des nappes comprenant des fibres solubles dans l'eau à une température inférieure ou égale à 30°C,
- la cavité contenant la composition contenant au moins un tensioactif moussant choisi parmi les acylaminoacides et leurs dérivés.

8. Article selon la revendication précédente, **caractérisé en ce que** les deux nappes de fibres sont des non-tissés.

9. Article selon la revendication précédente, **caractérisé en ce que** l'une des nappes est un non-tissé constitué de fibres solubles dans l'eau à une température inférieure ou égale à 30°C, et l'autre nappe est un non-tissé constitué de fibres insolubles dans l'eau.

10. Article selon l'une quelconque des revendications 6 à 9, **caractérisé en ce que** les deux nappes sont assemblées à leur périphérie.

11. Article selon la revendication précédente, **caractérisé en ce que** les nappes sont thermosoudées.

12. Article selon l'une quelconque des revendications 1 à 3, 6 ou 7, **caractérisé en ce que** le support est entièrement soluble dans l'eau.

13. Article selon l'une quelconque des revendications précédentes, **caractérisé en ce que** la quantité de tensioactif(s) dérivé(s) d'acylaminoacide représente de 2 à 100 % du poids total de la composition portée par le support.

14. Article selon l'une quelconque des revendications précédentes, **caractérisé en ce que** les acylaminoacides et leurs dérivés sont choisis parmi les sels d'acylalaninates, les sels d'acylglutamates, les sels d'acylaspartates, les sels d'acylglycinates, les sels d'acylsarcosinates, et leurs mélanges.

15. Article selon l'une quelconque des revendications précédentes, **caractérisé en ce que** les acylaminoacides et leurs dérivés ou la composition les contenant sont sous forme pulvérulente ou pâteuse.

16. Article selon l'une quelconque des revendications précédentes, **caractérisé en ce que** la composition contient un ou plusieurs tensioactifs moussants autres que les acylaminoacides et leurs dérivés.

17. Article selon l'une quelconque des revendications précédentes, **caractérisé en ce que** la composition portée par le support représente de 10 à 1000 % en poids par rapport au poids du support.

18. Article selon l'une quelconque des revendications précédentes, **caractérisé en ce qu'**il constitue un produit de nettoyage de la peau et/ou des cheveux ou un produit exfoliant pour la peau.

19. Composition pour application topique, obtenue par la dissolution dans l'eau, d'un article selon l'une quelconque des revendications 1 à 18.

20. Procédé cosmétique de nettoyage d'une matière kératinique, comportant :
- la formation d'une composition cosmétique par dissolution dans l'eau, d'un article selon l'une quelconque des revendications 1 à 17, et
- l'application de la composition ainsi formée sur la matière kératinique.

21. Ensemble comportant :
- un emballage,
- au moins un article selon l'une quelconque des revendications 1 à 17

## Claims

1. Item for topical application, comprising:
- a support in the form of at least one sheet comprising fibres that are water-soluble at a temperature less than or equal to 30°C, and
- a composition carried by the support, comprising at least one foaming surfactant chosen from acylamino acids and derivatives thereof.

2. Item according to Claim 1, **characterized in that** the fibres that are water-soluble at a temperature less than or equal to 30°C are prepared with polyvinyl alcohol.

3. Item according to Claim 1 or 2, **characterized in that** the sheet of fibres is a nonwoven.

4. Item according to any one of the preceding claims, **characterized in that** the sheet of fibres comprises water-insoluble fibres.

5. Item according to the preceding claim, **characterized in that** the amount of water-insoluble fibres is at most 40% by weight relative to the total weight of the fibres.

6. Item according to any one of the preceding claims, **characterized in that** the support comprises at least two sheets, at least one of which contains fibres that are water-soluble at a temperature less than or equal to 30°C.

7. Item according to any one of the preceding claims, **characterized in that** it comprises:
- at least two sheets that together define a cavity, at least one of the sheets comprising fibres that are water-soluble at a temperature less than or equal to 30°C,
- the cavity that contains the composition containing at least one foaming surfactant chosen from acylamino acids and derivatives thereof.

8. Item according to the preceding claim, **characterized in that** the two sheets of fibres are nonwovens.

9. Item according to the preceding claim, **characterized in that** one of the sheets is a nonwoven consisting of fibres that are water-soluble at a temperature of less than or equal to 30°C, and that the other sheet is a nonwoven consisting of water-insoluble fibres.

10. Item according to any one of Claims 6 to 9, **characterized in that** the two sheets are assembled at their periphery.

11. Item according to the preceding claim, **characterized in that** the sheets are heat-sealed.

12. Item according to any one of Claims 1 to 3, 6 or 7, **characterized in that** the support is entirely water-soluble.

13. Item according to any one of the preceding claims, **characterized in that** the amount of acylamino acid-derived surfactant(s) represents from 2 to 100% of the total weight of the composition carried by the support.

14. Item according to any one of the preceding claims, **characterized in that** the acylamino acids and derivatives thereof are chosen from acylalaninate salts, acylglutamate salts, acylaspartate salts, acylglycinate salts and acylsarcosinate salts, and mixtures thereof.

15. Item according to any one of the preceding claims, **characterized in that** the acylamino acids and derivatives thereof or the composition containing them are in pulverulent or pasty form.

16. Item according to any one of the preceding claims, **characterized in that** the composition contains one or more foaming surfactants other than the acylamino acids and derivatives thereof.

17. Item according to any one of the preceding claims, **characterized in that** the composition carried by the support represents from 10 to 1000% by weight relative to the weight of the support.

18. Item according to any one of the preceding claims, **characterized in that** it constitutes a cleansing product for the skin and/or the hair or an exfoliating product for the skin.

19. Composition for topical application, obtained by dissolving, in water, an item according to any one of Claims 1 to 18.

20. Cosmetic process for cleansing a keratin material, comprising:
- the formation of a cosmetic composition by dissolving, in water, an item according to any one of Claims 1 to 17, and
- the application of the composition thus formed to the keratin.material.

21. Assembly comprising:
- a packaging,
- at least one item according to any one of Claims 1 to 17.

## Patentansprüche

1. Artikel für die topische Anwendung, umfassend:
- einen Träger in der Form mindestens einer Lage, die bei einer Temperatur von 30 °C oder darunter in Wasserlöslich Fasern umfasst, und
- eine Zusammensetzung, die von dem Träger getragen wird und die mindestens einen schaumbildenden grenzflächenaktiven Stoff enthält, der unter den Acylaminosäuren und deren Derivaten ausgewählt ist.

2. Artikel nach Anspruch 1, **dadurch gekennzeichnet, dass** die bei einer Temperatur von 30 °C oder darunter in Wasser löslichen Fasern mit Polyvinylalkohol realisiert sind.

3. Artikel nach Anspruch 1 oder 2, **dadurch gekennzeichnet, dass** es sich bei der Lage aus Fasern um ein Nonwoven handelt.

4. Artikel nach einem der vorhergehenden Ansprüche, **dadurch gekennzeichnet, dass** die Lage aus Fasern in Wasser unlösliche Fasern umfasst.

5. Artikel nach dem vorhergehenden Anspruch, **dadurch gekennzeichnet, dass** der Mengenanteil der in Wasser unlöslichen Fasern höchstens 40 Gew.-%, bezogen auf das Gesamtgewicht der Fasern, beträgt.

6. Artikel nach einem der vorhergehenden Ansprüche, **dadurch gekennzeichnet, dass** der Träger mindestens zwei Lagen umfasst, von denen mindestens eine bei einer Temperatur von 30 °C oder darunter in Wasser lösliche Fasern enthält.

7. Artikel nach einem der vorhergehenden Ansprüche, **dadurch gekennzeichnet, dass** er umfasst:
- mindestens zwei Lagen, die zwischen sich einen Hohlraum erzeugen, wobei mindestens eine der Lagen bei einer Temperatur von 30 °C oder darunter in Wasser löslichen Fasern umfasst,
- wobei der Hohlraum die Zusammensetzung enthält, die mindestens einen schaumbildenden grenzflächenaktiven Stoff enthält, der unter den Acylaminosäuren und deren Derivaten ausgewählt ist.

8. Artikel nach dem vorhergehenden Anspruch, **dadurch gekennzeichnet, dass** es sich bei den beiden Lagen aus Fasern um Nonwovens handelt.

9. Artikel nach dem vorhergehenden Anspruch, **dadurch gekennzeichnet, dass** eine der Lagen ein Nonwoven ist, das aus bei einer Temperatur von 30 °C oder darunter in Wasser löslichen Fasern besteht, und die andere Lage ein Nonwoven ist, das aus in Wasser unlöslichen Fasern besteht.

10. Artikel nach einem der Ansprüche 6 bis 9, **dadurch gekennzeichnet, dass** die beiden Lagen an ihrem Rand zusammengefügt sind.

11. Artikel nach dem vorhergehenden Anspruch, **dadurch gekennzeichnet, dass** die Lagen warmverschweißt sind.

12. Artikel nach einem der Ansprüche 1 bis 3, 6 oder 7, **dadurch gekennzeichnet dass** der Träger in Wasser vollständig löslich ist.

13. Artikel nach einem der vorhergehenden Ansprüche, **dadurch gekennzeichnet, dass** der Mengenanteil des oder der von Acylaminosäuren abgeleiteten, grenzflächenaktiven Stoffe(s) 2 bis 100 Gew.-% des Gesamtgewichts der Zusammensetzung, die von dem Träger getragen wird, ausmacht.

14. Artikel nach einem der vorhergehenden Ansprüche, **dadurch gekennzeichnet, dass** die Acylaminosäuren und ihre Derivate unter den Salzen von Acylalaninaten, den Salzen von Acylglutamaten, den Salzen von Acylaspartaten, den Salzen von Acylglycinaten, den Salzen von Acylsarcosinaten und deren Gemischen ausgewählt sind.

15. Artikel nach einem der vorhergehenden Ansprüche, **dadurch gekennzeichnet, dass** die Acylaminosäuren und ihre Derivate oder die Zusammensetzung, die sie enthält, in Form von Pulver oder in pastöser Form vorliegen.

16. Artikel nach einem der vorhergehenden Ansprüche, **dadurch gekennzeichnet, dass** die Zusammensetzung einen oder mehrere schaumbildende grenzflächenaktive Stoffe enthält, die von den Acylaminosäuren und ihre Derivaten verschieden sind.

17. Artikel nach einem der vorhergehenden Ansprüche, **dadurch gekennzeichnet, dass** die Zusammensetzung, die von dem Träger getragen wird, 10 bis 1000 Gew.-%, gezogen auf das Gewicht des Trägers, ausmacht.

18. Artikel nach einem der vorhergehenden Ansprüche, **dadurch gekennzeichnet, dass** es sich um ein Produkt für die Reinigung der Haut und/oder der Haare oder ein exfoliatives Produkt für die Haut handelt.

19. Zusammensetzung für die topische Anwendung, die durch Auflösen eines Artikels nach einem der Ansprüche 1 bis 18 in Wasser erhalten wird.

20. Kosmetisches Verfahren für die Reinigung einer Keratinsubstanz, das umfasst:
- es wird eine kosmetische Zusammensetzung gebildet, indem ein Artikel nach einem der Ansprüche 1 bis 17 in Wasser aufgelöst wird, und
- die auf diese Weise gebildete Zusammensetzung wird auf die Keratinsubstanz aufgebracht.

21. Einheit, umfassend:
- eine Verpackung,
- mindestens einen Artikel nach einem der Ansprüche 1 bis 17.
